# EUROPEAN PATENT APPLICATION

(11) **EP 4 782 430 A1**
(43) Date of publication of application: **29.07.2026**
(21) Application number: 24868271.8
(22) Date of filing: 18.09.2024
(51) Int. Cl.: C07C 211/64, C07F 5/02, C08F 2/06, C08F 4/12, C08F 10/00, C08F 36/00

(54) **NOVEL BORATE COMPOUND SOLUBLE IN ALIPHATIC HYDROCARBON SOLVENT**

(30) Priority: 21.09.2023 JP 2023156460
(71) Applicant: AGC Inc., Chiyoda-ku Tokyo 100-8405 (JP)
(72) Inventor: SAWAGUCHI, Masanori, Tokyo 100-8405 (JP)
(74) Representative: TBK
(86) International application number: PCT/JP2024/033234
(87) International publication number: WO 2025/063194

(57) **Abstract**

The present invention aims to provide a borate compound which is soluble in aliphatic hydrocarbon solvents, capable of maintaining a homogeneous solution state even at low temperatures, and useful as a cocatalyst for the solution polymerization of olefin and diene.

The present invention relates to a borate compound represented by the following formula (1): 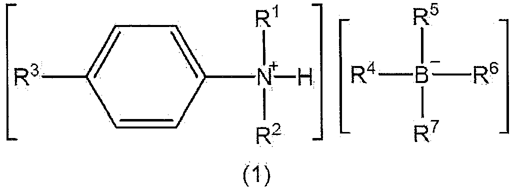 wherein each symbol is as defined in the specification, a production method thereof, and use thereof as a cocatalyst for the polymerization of olefin and diene.

## Description

### [Technical Field]

The present invention relates to a borate compound which is soluble in aliphatic hydrocarbon solvents, capable of maintaining a homogeneous solution state even at low temperatures, and useful as a cocatalyst for the polymerization of olefin and diene, and a production method thereof.

### [Background Art]

Many reports have been conventionally made on the use of metallocene compound and non-metallocene type metal complex catalysts such as diimine complex, phenoxy complex, and the like as catalysts for the polymerization of olefins and dienes. As cocatalysts used for stabilizing the cationic active species of these metal complex catalysts, alkylaluminium, aluminoxanes such as methylaluminoxane (MAO), and the like, Broensted acid salts such as ammonium borate and the like, and Lewis acid salts such as triphenylcarbenium borate and the like are used (Non-Patent Literature 1).

In the catalyst activation reaction by the aforementioned Broensted acid salt, the leaving group on the metal complex catalyst is protonated and eliminated from the metal complex catalyst to generate a cationic active species of the metal complex catalyst, whereby non-coordinating anions derived from Broensted acid salt stabilize the active species. As the Broensted base constituting the Broensted acid salt, various borate compounds such as tetrakis(pentafluorophenyl)borate, which is a non-coordinating anion, and the like have been reported (Non-Patent Literature 1) and, as the Broensted acid, Broensted acids containing nitrogen, phosphorus, oxygen, and/or sulfur are known (Patent Literature 1).

As the aforementioned Broensted acid salts, Broensted acid salts (ammonium borates) containing nitrogen such as dimethylanilinium tetrakis(pentafluorophenyl)borate, tri(n-butyl) ammonium tetrakis(pentafluorophenyl)borate, methylpyrrolidinium tetrakis(pentafluorophenyl)borate, and the like are known (Patent Literature 2). In the catalyst activation reactions by these ammonium borates, amine compounds are generated due to the loss of proton in the protonation stage. Such amine compound may interact with the cationic active species of the metal complex catalyst, in which case the compound is feared to adversely affect the polymerization reaction.

In addition, as a solvent used for polymerization, a nonpolar hydrocarbon solvent is used. In particular, from the aspects of odor and toxicity, switching to aliphatic hydrocarbon solvents such as n-hexane and the like from aromatic hydrocarbon solvents such as toluene and the like is progressing.

However, it is known that general tetrakis(pentafluorophenyl)borate compounds are hardly soluble in aromatic hydrocarbon solvents such as toluene and the like, or that even if dissolved, they are separated to form two liquid-liquid phases of a concentrated phase in which the borate compound is dissolved and a dilute phase in which it is not dissolved (Patent Literature 3).

Since tetrakis(pentafluorophenyl)borate compounds are hardly soluble in aliphatic hydrocarbon solvents such as n-hexane, n-heptane, and the like, a compound soluble in aliphatic hydrocarbon solvents is desired and has been proposed (Patent Literature 4). Di(octadecyl)methylammonium tetrakis(pentafluorophenyl)borate and bis(hydrogenated tallow)methylammonium tetrakis(pentafluorophenyl)borate described in Patent Literature 4 are useful as compounds soluble in hydrocarbon solvents.

However, when di(octadecyl)methylammonium tetrakis(pentafluorophenyl)borate or bis(hydrogenated beef tallow alkyl)methylammonium tetrakis(pentafluorophenyl)borate described in Patent Literature 4 is used, since trialkylamine generated after a catalyst activation reaction has nucleophilicity, it is feared that it becomes a catalyst poison in the polymerization reaction of olefins or dienes.

In addition, Patent Literature 5 describes use of a compound represented by the following formula (AI):

[Chem. 1] [R¹R²R³EH] _{d}⁺ [M^{k+}Qₙ]^{d-} (AI)

wherein each symbol is as defined in Patent Literature 5, as an activator for olefin polymerization (hereinafter referred to as a cocatalyst in the present specification), and describes that the solubility of the compound in an aliphatic hydrocarbon solvent at 25°C increases as the number of aliphatic carbon atoms in the cationic group (i.e., ammonium or phosphonium) increases. However, Patent Literature 5 does not disclose the solubility of the compound in an aliphatic hydrocarbon solvent at low temperatures.

### [Citation List]

### [Patent Literature]

[Patent Literature 1]
   US Patent No. 5132380
[Patent Literature 2]
   WO 2010/014344
[Patent Literature 3]
   JP 2018-104335 A
[Patent Literature 4]
   JP 2000-507157 A
[Patent Literature 5]
   JP 2021-522243 A

### [Non Patent Literature]

[Non Patent Literature 1]
Chem. Rev. 2000, 100, 1391-1434

### [Summary of Invention]

### [Technical Problem]

Generally, borate compounds, which are cocatalysts for the solution polymerization of olefins and dienes, are obtained in an oily form and are therefore not easy to handle and measure as a single substance. Therefore, it is necessary that borate compounds are generally dissolved in a solvent used for solution polymerization and stored as a homogeneous solution, and then diluted and measured at the time of use. However, as described above, it is known that borate compounds generally have extremely low solubility in aliphatic hydrocarbon solvents. Furthermore, the present inventors have encountered difficulties such as separation into two phases and precipitation of solids when the borate compound described in Patent Document 5, which is soluble in aliphatic hydrocarbon solvents at 25°C, is stored as a solution in an aliphatic hydrocarbon solvent in winter season when outdoor temperature is low or at low temperatures. In view of those conventional techniques, the present invention aims to provide a borate compound, which is soluble in hydrocarbon solvents, capable of maintaining a homogeneous solution state even at low temperatures, and useful as a cocatalyst for the solution polymerization reaction of olefins and dienes, and an industrial production method thereof.

### [Solution to Problem]

The present inventors have conducted intensive studies and found that a compound represented by the following formula (1): 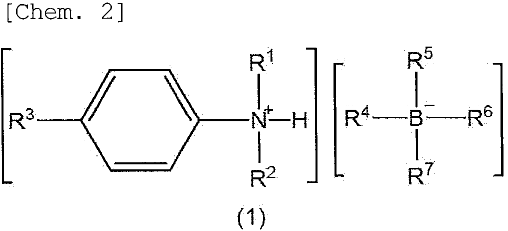
wherein R¹ and R² are each independently a linear C₁₆₋₂₀ alkyl group; R³ is a linear C₃₋₈ alkyl group; and
R⁴, R⁵, R⁶, and R⁷ are each independently a C₆₋₁₄ aryl group substituted by one or more fluorine atoms or fluoro C₁₋₄ alkyl groups (hereinafter to be also referred to as "the compound of the present invention") is soluble in aliphatic hydrocarbon solvents such as n-hexane, methylcyclohexane, and the like, can be stored stably in a homogeneous solution state even under low temperatures, and can be used as it is when in use, and thus is useful as a cocatalyst for the solution polymerization reaction of olefin and diene, and completed the present invention.

One embodiment of the present invention is, for example, as follows.
[1] A compound represented by the formula (1): 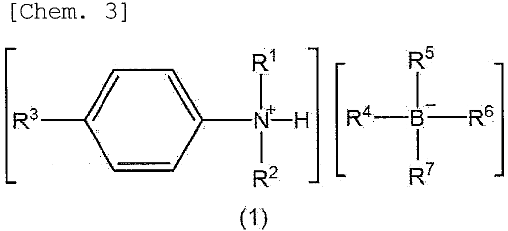
   wherein R¹ and R² are each independently a linear C₁₆₋₂₀ alkyl group; R³ is a linear C₃₋₈ alkyl group; and
   R⁴, R⁵, R⁶ and R⁷ are each independently a C₆₋₁₄ aryl group substituted by one or more fluorine atoms or fluoro C₁₋₄ alkyl groups.
[2] The compound of the aforementioned [1], wherein R¹ and R² are each independently a linear C₁₆₋₁₈ alkyl group, and R³ is a linear C₄₋₆ alkyl group.
[3] The compound of the aforementioned [1], wherein R⁴, R⁵, R⁶ and R⁷ are each independently a phenyl group, a 1-naphthyl group, or a 2-naphthyl group, each of which is substituted by one or more fluorine atoms or trifluoromethyl groups.
[4] The compound of the aforementioned [1] or [2], wherein all of R⁴, R⁵, R⁶ and R⁷ are pentafluorophenyl groups, 2,3,4,5,6,7,8-heptafluoro-1-naphthyl groups, or 1,3,4,5,6,7,8-heptafluoro-2-naphthyl groups.
[5] The compound according to any one of the aforementioned [1] to [4], wherein the compound exhibits a solubility of 3 mM or more in n-hexane within the range of -15°C to 25°C and a solubility of 300 mM or more in methylcyclohexane within the range of -15°C to 25°C.
[6] A n-hexane solution of the compound according to any one of the aforementioned [1] to [4], wherein 3 mM or more of the compound is contained within the range of -15°C to 25°C.
[7] A methylcyclohexane solution of the compound according to any one of the aforementioned [1] to [4], wherein 300 mM or more of the compound is contained within the range of -15°C to 25°C.
[8] A cocatalyst for solution polymerization of at least one kind of monomer selected from the group consisting of an olefin and a diene, consisting of the compound of any of the aforementioned [1] to [4].
[9] A method for producing a polymer, comprising solution polymerizing at least one kind of monomer selected from the group consisting of an olefin and a diene by using the compound of any of the aforementioned [1] to [4] as a cocatalyst.
[10] A method for storing the compound of any of the aforementioned [1] to [4], comprising dissolving the compound in n-hexane or methylcyclohexane, and storing same at -15°C to 0°C while maintaining a homogeneous solution state.
[11] The method for producing the compound of the aforementioned [1], comprising a step of reacting a compound represented by the formula (2): 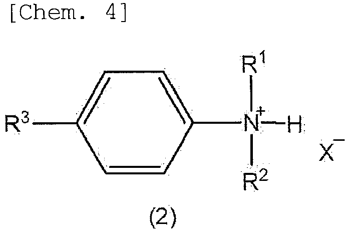
   wherein R¹ and R² are each independently a linear C₁₆₋₂₀ alkyl group; R³ is a linear C₃₋₈ alkyl group; and
   X is a halogen atom, and a compound represented by the formula (3): 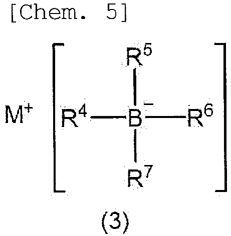
      wherein R⁴, R⁵, R⁶, and R⁷ are each independently a C₆₋₁₄ aryl group substituted by one or more fluorine atoms or fluoro C₁₋₄ alkyl groups; and
      M is an alkali metal, in a mixed solvent of methylcyclohexane and water.

### [Advantageous Effects of Invention]

Generally, in the technical field of solution polymerization of olefins and dienes, aromatic hydrocarbon solvents such as toluene and the like are frequently used as solvents or co-solvents in consideration of the solubility of catalyst and the like. However, it is desirable to reduce the amount of aromatic hydrocarbon solvents or not use them not only from the viewpoint of odor and toxicity but also from the viewpoint of removal costs. According to the present invention, a novel borate compound capable of maintaining a homogeneous solution state in an aliphatic hydrocarbon solvent, particularly n-hexane, methylcyclohexane, or a mixed solvent thereof, even at low temperatures, can be provided without using any aromatic hydrocarbon solvent. The compound of the present invention can maintain a homogeneous solution state in aliphatic hydrocarbon solvents even at low temperatures, and thus affords the advantage that the compound of the present invention can be stably stored in a diluted solution state that facilitates handling and measurement, and can be used as it is when in use as a cocatalyst for solution polymerization reaction of olefin and diene. According to the present invention, moreover, a convenient production method of the compound of the present invention can also be provided.

### [Description of Embodiments]

The definitions of the terms and respective symbols used in the present specification are explained below.

In the present specification, the "halogen atom" means a fluorine atom, a chlorine atom, a bromine atom, or an iodine atom.

In the present specification, the "linear C₁₆₋₂₀ alkyl (group)" means a linear alkyl group having a carbon atom number of 16 to 20. Examples thereof include hexadecyl, octadecyl, nonadecyl, eicosyl, and the like. Among them, a linear C₁₆₋₁₈ alkyl group is preferred.

In the present specification, the "linear C₃₋₈ alkyl (group)" means a linear alkyl group having a carbon atom number of 3 to 8. Examples thereof include n-propyl, n-butyl, n-pentyl, n-hexyl, n-heptyl, n-octyl, and the like. Among them, a linear C₄₋₆ alkyl group is preferred.

In the present specification, the "C₁₋₄ alkyl (group)" means a linear or branched chain alkyl group having a carbon atom number of 1 to 4. Examples thereof include methyl, ethyl, n-propyl, isopropyl, n-butyl, isobutyl, sec-butyl, tert-butyl, and the like.

In the present specification, the "fluoro C₁₋₄ alkyl (group)" means the aforementioned "C₁₋₄ alkyl" group in which one or more hydrogen atoms are substituted by fluorine atom(s). Specific examples thereof include difluoromethyl, trifluoromethyl, 2-fluoroethyl, 2,2-difluoroethyl, 2,2,2-trifluoroethyl, pentafluoroethyl, 2,2,3,3-tetrafluoropropyl, 3,3,3-trifluoropropyl, 4,4,4-trifluorobutyl, and the like.

In the present specification, the "alkali metal" means a metal element belonging to Group I (Group IA) of the periodic table, and includes lithium, sodium, potassium, rubidium, cesium, and the like. Among them, lithium or sodium is preferred.

In the present specification, the "hydrocarbon solvent" means solvents including aromatic hydrocarbon solvents and/or aliphatic hydrocarbon solvents.

In the present specification, examples of the "aromatic hydrocarbon solvent" include benzene, toluene, xylene, and the like.

In the present specification, examples of the "aliphatic hydrocarbon solvent" include n-hexane, isohexane, n-heptane, n-octane, cyclohexane, methylcyclohexane, a mixed solvent thereof, and the like. Among them, n-hexane, methylcyclohexane, or a mixed solvent thereof is preferred.

In the present specification, "soluble in aliphatic hydrocarbon solvents" means that the compound of the present invention dissolves in an aliphatic hydrocarbon solvent such as n-hexane or methylcyclohexane, and the like at a specific concentration at a specific temperature to form a transparent, homogeneous solution. Examples of specific preferred embodiment include that the compound of the present invention dissolves in n-hexane at a concentration of at least 3 mM, preferably 5 mM or more, within the range of -15°C to 25°C (at any temperature within this range) to form a transparent, homogeneous solution, that the compound of the present invention dissolves in methylcyclohexane at a concentration of at least 300 mM, preferably 350 mM or more, more preferably 400 mM or more, within the range of -15°C to 25°C (at any temperature within this range) to form a transparent, homogeneous solution, and the like.

In the present specification, "under low temperature" means a temperature lower than the ordinary temperature. The upper limit is not particularly limited as long as it is lower than 15°C, but is generally 10°C or lower, preferably 5°C or lower, more preferably 0°C or lower. The lower limit is generally -20°C or higher, preferably -15°C or higher.

In the present specification, "ordinary temperature" refers to the ordinary temperature (15°C to 25°C) as defined in the General Rules of the Japanese Pharmacopoeia, 18th Edition.

In the present specification, "solubility within the range of -15°C to 25°C" means the molar concentration of the compound of the present invention that can maintain a transparent, homogeneous solution when a solution of the compound of the present invention dissolved in an aliphatic hydrocarbon solvent at 25°C is cooled to any temperature within the range of -15°C to 25°C, that is, the concentration (mM) of a saturated solution of the compound of the present invention in an aliphatic hydrocarbon solvent at any temperature within the range of -15°C to 25°C.

In the present specification, indication of "Y°C to Z°C" (Y and Z are any numbers) means "Y°C or higher and Z°C or lower", unless otherwise specified.

In the present specification, "homogeneous solution state" means a transparent state in which the compound of the present invention is homogeneously dissolved in an aliphatic hydrocarbon solvent without precipitating as a solid or undergoing phase separation (e.g., separation into two phases).

In the present specification, storage in the "storage method" includes maintaining a stationary state for a certain period of time, and also includes moving not only in a stationary state.

In the present specification, "solution polymerization" means a polymerization reaction performed in a solvent such as an organic solvent and the like. Therefore, solution polymerization is performed always in a solution state, i.e., a homogeneous solution state, from a mixed solution state of the monomer and solvent to during and after completion of the polymerization reaction.

### (Compound of the present invention)

The compound of the present invention is described below.

The compound of the present invention is a compound represented by the following formula (1): 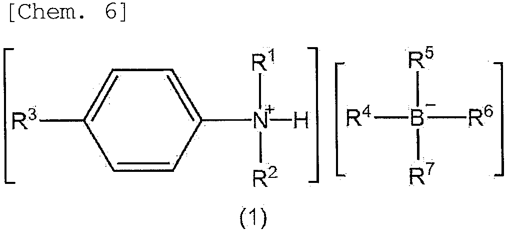
wherein R¹ and R² are each independently a linear C₁₆₋₂₀ alkyl group; R³ is a linear C₃₋₈ alkyl group; and
R⁴, R⁵, R⁶ and R⁷ are each independently a C₆₋₁₄ aryl group substituted by one or more fluorine atoms or fluoro C₁₋₄ alkyl groups (hereinafter also to be referred to as "compound (1)").

Preferred embodiments of compound (1) are described in the following.

In the following, each group of compound (1) is described.

R¹ and R² are preferably each independently a linear C₁₆₋₁₈ alkyl group, and R³ is a linear C₄₋₆ alkyl group.

R⁴, R⁵, R⁶, and R⁷ are preferably each independently a phenyl group, a 1-naphthyl group, a 2-naphthyl group, a 2-biphenylyl group, a 3-biphenylyl group, a 4-biphenylyl group, a 1-anthryl group, a 2-anthryl group, a 9-anthryl group, a 3-phenanthryl group, or a 9-phenanthryl group, each substituted by one or more fluorine atoms or fluoro C₁₋₄ alkyl groups (e.g., trifluoromethyl groups), more preferably each independently a phenyl group, a 1-naphthyl group, or a 2-naphthyl group, each substituted by one or more fluorine atoms or trifluoromethyl groups, particularly preferably R⁴, R⁵, R⁶, and R⁷ are all the same and are pentafluorophenyl groups, 2,2',3,3',4',5,5',6,6'-nonafluoro-4-(1,1'-biphenylyl) groups, 2,3,4,5,6,7,8-heptafluoro-1-naphthyl groups, or 1,3,4,5,6,7,8-heptafluoro-2-naphthyl groups.

As preferred compound (1), the following compounds can be mentioned.

### [Compound (1A)]

Compound (1) of the aforementioned formula (1), wherein
R¹ and R² are each independently a linear C₁₆₋₂₀ alkyl group, R³ is a linear C₃₋₈ alkyl group, and
R⁴, R⁵, R⁶ and R⁷ are each independently a phenyl group, a 1-naphthyl group, a 2-naphthyl group, a 2-biphenylyl group, a 3-biphenylyl group, a 4-biphenylyl group, a 1-anthryl group, a 2-anthryl group, a 9-anthryl group, a 3-phenanthryl group, or a 9-phenanthryl group, each of which is substituted by one or more fluorine atoms or fluoro C₁₋₄ alkyl groups (e.g., trifluoromethyl groups).

### [Compound (1B)]

Compound (1) of the aforementioned formula (1), wherein
R¹ and R² are each independently a linear C₁₆₋₂₀ alkyl group, R³ is a linear C₃₋₈ alkyl group, and
R⁴, R⁵, R⁶ and R⁷ are each independently a phenyl group, a 1-naphthyl group, or a 2-naphthyl group, each of which is substituted by one or more fluorine atoms or trifluoromethyl groups.

### [Compound (1C)]

Compound (1) of the aforementioned formula (1), wherein
R¹ and R² are each independently a linear C₁₆₋₂₀ alkyl group,
R³ is a linear C₃₋₈ alkyl group, and
R⁴, R⁵, R⁶ and R⁷ are all the same and are pentafluorophenyl groups, 2,2',3,3',4',5,5',6,6'-nonafluoro-4-(1,1'-biphenylyl) groups, 2,3,4,5,6,7,8-heptafluoro-1-naphthyl groups, or 1,3,4,5,6,7,8-heptafluoro-2-naphthyl groups (preferably pentafluorophenyl group).

### [Compound (1D)]

Compound (1) of the aforementioned formula (1), wherein
R¹ and R² are each independently a linear C₁₆₋₁₈ alkyl group, R³ is a linear C₄₋₆ alkyl group, and
R⁴, R⁵, R⁶ and R⁷ are each independently a phenyl group, a 1-naphthyl group, a 2-naphthyl group, a 2-biphenylyl group, a 3-biphenylyl group, a 4-biphenylyl group, a 1-anthryl group, a 2-anthryl group, a 9-anthryl group, a 3-phenanthryl group, or a 9-phenanthryl group, each of which is substituted by one or more fluorine atoms or fluoro C₁₋₄ alkyl groups (e.g., trifluoromethyl group).

### [Compound (1E)]

Compound (1) of the aforementioned formula (1), wherein
R¹ and R² are each independently a linear C₁₆₋₁₈ alkyl group, R³ is a linear C₄₋₆ alkyl group, and
R⁴, R⁵, R⁶ and R⁷ are each independently a phenyl group, a 1-naphthyl group, or a 2-naphthyl group, each of which is substituted by one or more fluorine atoms or trifluoromethyl groups.

### [Compound (1F)]

Compound (1) of the aforementioned formula (1), wherein
R¹ and R² are each independently a linear C₁₆₋₁₈ alkyl group, R³ is a linear C₄₋₆ alkyl group, and
R⁴, R⁵, R⁶ and R⁷ are all the same and are pentafluorophenyl groups, 2,2',3,3',4',5,5',6,6'-nonafluoro-4-(1,1'-biphenylyl) groups, 2,3,4,5,6,7,8-heptafluoro-1-naphthyl groups, or 1,3,4,5,6,7,8-heptafluoro-2-naphthyl groups (preferably pentafluorophenyl group).

Specific preferred examples of compound (1) include N,N-dihexadecyl-4-butylanilinium tetrakis(pentafluorophenyl)borate (compound (1-1)), N,N-dioctadecyl-4-butylanilinium tetrakis(pentafluorophenyl)borate (compound (1-2)), and the like.

The compound of the present invention is soluble in aliphatic hydrocarbon solvents, particularly n-hexane, methylcyclohexane, or mixed solvents thereof, and can maintain a homogeneous solution state even at low temperatures. This affords the advantage that the compound of the present invention can be stably stored in a solution state that facilitates handling and measurement. Therefore, the compound of the present invention is useful as a cocatalyst for the homogeneous solution polymerization of olefins and dienes.

### (Storage method of the compound of the present invention)

A method for storing the compound of the present invention (hereinafter to be also referred to as "the storage method of the present invention") is described below.

The storage method of the present invention is characterized in that the compound of the present invention (compound (1)) is dissolved in an aliphatic hydrocarbon solvent (e.g., n-hexane, methylcyclohexane, or a mixed solvent thereof), and stored at low temperature for a certain period of time while maintaining a homogeneous solution state. It is possible to confirm by visual observation that the mixture of the compound of the present invention and the aliphatic hydrocarbon solvent is in a homogeneous solution state.

The lower limit of the concentration of compound (1) during storage, i.e., solubility, can be determined based on the concentration (mM) of a saturated solution of compound (1) in an aliphatic hydrocarbon solvent at the lowest storage temperature, specifically 20°C or -15°C.

A preferred one embodiment of the storage method of the present invention is a storage method characterized by dissolving compound (1) in n-hexane at a concentration of at least 3 mM, and storing same at -15°C to 0°C for a certain period of time while maintaining a homogeneous solution state.

Another preferred embodiment of the storage method of the present invention is a storage method characterized by dissolving compound (1) in methylcyclohexane at a concentration of at least 300 mM, and storing same at -15°C to 0°C for a certain period of time while maintaining a homogeneous solution state.

### (Production method of the compound of the present invention)

The production method of the compound of the present invention (compound (1)) (hereinafter to be also referred to as "the production method of the present invention") is described below.

The production method of the present invention is a production method of compound (1) and characteristically includes a step of reacting a compound represented by the following formula (2): 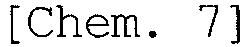 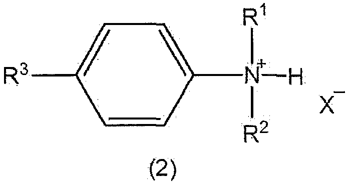
wherein each symbol has the same meaning as defined above (hereinafter also to be referred to as "compound (2)") and a compound represented by the formula (3): 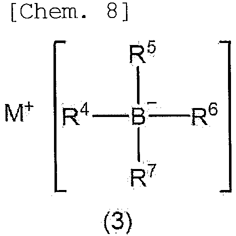
wherein each symbol has the same meaning as defined above (hereinafter also to be referred to as "compound (3)") in a mixed solvent of methylcyclohexane and water (hereinafter also to be referred to as "step A"). Specifically, the production method described in the below-mentioned Production Example can be mentioned.

Examples of compound (2) used as a raw material in this production method include N,N-dihexadecylaniline hydrochloride, N,N-diheptadecylaniline hydrochloride, N,N-dioctadecylaniline hydrochloride, N,N-dinonadecylaniline hydrochloride, N,N-dieicosylaniline hydrochloride and the like.

The production method of compound (2) is not particularly limited and includes, for example, a step of reacting a compound represented by the following formula (4): 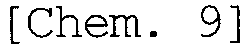 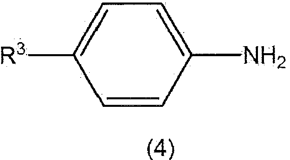
wherein each symbol has the same meaning as defined above (hereinafter also to be referred to as "compound (4)") with alkyl halide (R¹X' and R²X') wherein X' is a halogen atom, and other symbols have the same meaning as defined above, in a solvent in the presence of a base to obtain a compound represented by the following formula (5): 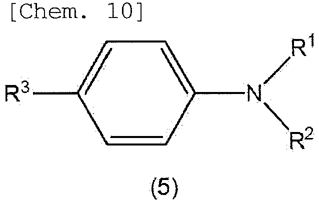
wherein each symbol has the same meaning as defined above (hereinafter also to be referred to as "compound (5)") (hereinafter also to be referred to as "step 1"), and a step of reacting the aforementioned compound (5) with hydrogen chloride in a solvent to convert same into the aforementioned compound (2) (hereinafter also to be referred to as "step 2").

### (Step 1)

This step can be performed in a solvent that does not affect the reaction. The reaction solvent is not particularly limited, and examples include ethers such as diethyl ether, tetrahydrofuran, dioxane, and the like; aromatic hydrocarbons such as toluene, xylene, and the like; nitriles such as acetonitrile and the like; and amides such as N,N-dimethylformamide, N,N-dimethylacetamide, and the like. Of these, tetrahydrofuran is preferred.

Examples of the base include inorganic bases such as sodium hydride, potassium carbonate, sodium hydroxide, potassium hydroxide, and the like; and organic bases such as triethylamine, pyridine, N,N-diisopropylethylamine, and the like. Of these, sodium hydride is preferred.

The amount of the base to be used is generally 2 to 5 moles, preferably 2.2 to 3 moles, per mole of compound (4).

The amount of alkyl halide to be used is generally 1 to 1.5 moles, preferably 1.1 to 1.3 moles, for each of R¹X' and R²X' per mole of compound (4). When R¹ and R² are the same, the total amount of R¹X' and R²X' to be used is generally 2 to 3 moles, preferably 2.2 to 2.6 moles.

The reaction temperature is generally 60°C to 130°C, preferably 70°C to 90°C, more preferably the reflux temperature of the reaction solvent. The reaction time is generally 3 to 24 hr.

### (Step 2)

This step can be performed in a solvent that does not affect the reaction. The reaction solvent is not particularly limited, and examples include aliphatic hydrocarbon solvents such as n-hexane, n-heptane and the like. Of these, n-hexane is preferred.

Hydrogen chloride is not particularly limited and, for example, a hydrogen chloride/diethyl ether solution or a hydrogen chloride/ethyl acetate solution, both of which are readily available as commercially available products, can be used.

The amount of hydrogen chloride to be used is generally 1 to 2 moles, preferably 1.1 to 1.5 moles, per mole of compound (5).

The reaction temperature is generally ordinary temperature (15°C to 25°C), preferably room temperature (20°C to 25°C), and the reaction time is generally 1 to 4 hr.

In this production method, examples of compound (3) used as a raw material include lithium tetrakis(pentafluorophenyl)borate, sodium tetrakis(pentafluorophenyl)borate, lithium tetrakis(nonafluoro[1,1'-biphenyl]-4-yl)borate, sodium tetrakis(nonafluoro[1,1'-biphenyl]-4-yl)borate, lithium tetrakis(heptafluoro-2-naphthyl)borate, sodium tetrakis(heptafluoro-2-naphthyl)borate, lithium [3,5-bis(trifluoromethyl)phenyl]borate, sodium [3,5-bis(trifluoromethyl)phenyl]borate, diethyl ether complexes thereof, and the like.

Compound (3) to be used may be a commercially available product or a purified product, or may be prepared by known methods (see, for example, Angew. Chem. Int. Ed., 2009, 48(40), 7444-7447).

### (Step A)

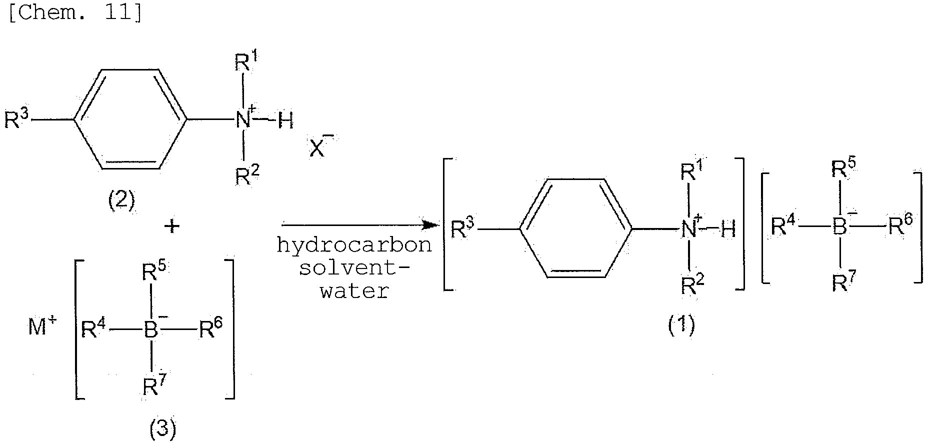

This step can be performed in a hydrocarbon solvent-water mixed solvent (two-phase system). The hydrocarbon solvent to be used is not particularly limited, and examples include aromatic hydrocarbon solvents such as toluene, xylene, and the like, or aliphatic hydrocarbon solvents such as n-hexane, cyclohexane, methylcyclohexane, and the like. Among these, aliphatic hydrocarbon solvents such as methylcyclohexane and the like are preferred, since the solution after work-up can be used as it is in the solution polymerization reaction. The mixing ratio (v/v) of hydrocarbon solvent and water can be appropriately determined depending on the kind of the hydrocarbon solvent used. Hydrocarbon solvent:water is generally 1:1 to 10:1, preferably 1:1 to 5:1.

The reaction temperature is generally ordinary temperature (15°C to 25°C), preferably room temperature (20°C to 25°C). The reaction time is generally 1 to 4 hr.

In this step, after completion of the reaction, the organic layer is washed with water, the hydrocarbon solvent is evaporated under reduced pressure, and the hydrocarbon solvent is added to the residue and evaporated again under reduced pressure. This process is repeated three times, and the obtained residue is dissolved in an aliphatic hydrocarbon solvent for a work-up to obtain a solution of the compound of the present invention in an aliphatic hydrocarbon solvent. By quantifying the concentration of the compound of the present invention contained in the solution, the solution can be stored at low temperatures in a homogeneous, transparent solution state, and can be conveniently used as a cocatalyst for the solution polymerization of olefins and dienes when in use.

The method for quantifying the concentration (solubility) of the compound of the present invention contained in an aliphatic hydrocarbon solvent solution of the compound of the present invention is not particularly limited, and the concentration can be quantified, for example, by quantitative analysis by NMR using an internal standard.

The compound of the present invention is soluble in aliphatic hydrocarbon solvents and can be stably stored while maintaining a homogeneous solution state even at low temperatures. Therefore, the compound of the present invention and/or an aliphatic hydrocarbon solvent solution of the compound of the present invention (e.g., n-hexane solution, methylcyclohexane solution) are/is useful as a cocatalyst for the solution polymerization of olefins and dienes, which is easy to quantify and handle.

The present invention includes a production method of a polymer by solution polymerizing at least one kind of monomer selected from the group consisting of an olefin and a diene, by using the compound of the present invention as a cocatalyst.

Production of a polymer by using the compound of the present invention as a cocatalyst can be specifically performed according to, for example, the method described in the below-mentioned Experimental Example 2.

### [Example]

The present invention is specifically explained in detail in the following by referring to Production Examples, Examples, and Experimental Examples; however, the present invention is not limited to those Production Examples and Examples alone. % means mol/mol% for yield and wt% for others unless particularly indicated. The room temperature is 20°C to 25°C.

For the analysis, the following instrument was used.

¹H-NMR and ¹⁹F-NMR: ECZ400S FT-NMR manufactured by JEOL Ltd.

Unless particularly indicated, the solvents and reagents used in the following Production Examples and Examples were purchased from distributors such as Sigma-Aldrich, Tokyo Chemical Industry Co., Ltd., FUJIFILM Wako Pure Chemical Corporation, JUNSEI CHEMICAL CO., LTD., KANTO CHEMICAL CO., INC., Combi-Blocks, Inc., and the like. The deuterated solvents used for NMR measurement were purchased from Cambridge Isotope Laboratories.

### [Production Example 1]

### Synthesis of N,N-dihexadecyl-4-butylaniline hydrochloride

(1) To sodium hydride (content 66.4 wt%, 1.26 g, 33.8 mmol) was added tetrahydrofuran (10.7 g), and a solution of 4-butylaniline (2.0 g, 13.4 mmol) in tetrahydrofuran (5.6 g) was added dropwise while heating with refluxing at 75°C in an oil bath. Then, a solution of 1-bromohexadecane (8.2 g, 26.9 mmol) in tetrahydrofuran (17.8 g) was added dropwise to the reaction mixture, and the mixture was continuously heated under refluxed for 20 hr. The reaction mixture was cooled, and a mixed solution of water (0.4 g) and tetrahydrofuran (1.9 g) was added. After the hydrogen gas generation subsided, water (10.0 g) was added thereto, and the mixture was separated. The organic layer was concentrated under reduced pressure, the residue was dissolved in n-hexane (53.6 g), and the solution was washed with water, concentrated under reduced pressure, and dried to give N,N-dihexadecyl-4-butylaniline (8.2 g).
   ¹H NMR(CDCl₃) δ: 7.01 (d, J=8.7Hz, 2H), 6.57 (d, J=8.7Hz, 2H), 3.20 (t, J=7.5Hz, 4H), 2.49 (t, J=7.8Hz, 2H), 1.48-1.64 (m, 4H), 1.09-1.45 (m, 56H), 0.78-0.97 (m, 9H).
(2) N,N-Dihexadecyl-4-butylaniline (8.2 g, 13.7 mmol) obtained in the above-mentioned (1) was dissolved in n-hexane (27.2 g), and 4 mol/L hydrogen chloride/ethyl acetate solution (3.4 g, 15.4 mmol) was added dropwise with stirring, after which n-hexane (19.6 g) was added, and the mixture was stirred at room temperature for 1 hr. The resulting solid was collected by filtration, washed with n-hexane (11.0 g), and dried under reduced pressure to give the title compound (7.2 g, 11.3 mmol). ¹H NMR(CDCl₃) δ: 13.70 (s, 1H), 7.60 (d, J=7.9Hz, 2H), 7.30 (d, J=8.6Hz, 2H), 3.35-3.53 (m, 2H), 3.03-3.24 (m, 2H), 2.65 (t, J=7.6Hz, 2H), 1.90-2.12 (m, 2H), 1.53-1.68 (m, 2H), 0.99-1.46 (m, 56H), 0.94 (t, J=7.3Hz, 3H), 0.88 (t, J=7.0Hz, 6H).

### [Production Example 2]

### Synthesis of N,N-dioctadecyl-4-butylaniline hydrochloride

(1) To sodium hydride (content 66.4 wt%, 1.26 g, 33.8 mmol) was added tetrahydrofuran (10.7 g), and a solution of 4-butylaniline (2.0 g, 13.4 mmol) in tetrahydrofuran (5.6 g) was added dropwise while heating with refluxing at 75°C in an oil bath. Then, to the reaction mixture was added dropwise a solution of 1-bromooctadecane (9.8 g, 29.4 mmol) in tetrahydrofuran (17.8 g), and the mixture was continuously heated under refluxed for 21 hr. The reaction mixture was cooled, and a mixed solution of water (0.5 g) and tetrahydrofuran (1.8 g) was added. After the hydrogen gas generation subsided, water (10.0 g) was added, and the mixture was separated. The organic layer was concentrated under reduced pressure, the residue was dissolved in n-hexane (53.6 g), and the solution was washed with water, concentrated under reduced pressure, and dried to give N,N-dioctadecyl-4-butylaniline (9.7 g).
   ¹H NMR(CDCl₃) δ: 7.01 (d, J=8.7Hz, 2H), 6.57 (d, J=8.7Hz, 2H), 3.21 (t, J=7.5Hz, 4H), 2.49 (t, J=7.5Hz, 2H), 1.47-1.65 (m, 4H), 1.01-1.46 (m, 64H), 0.79-0.98 (m, 9H).
(2) N,N-Dioctadecyl-4-butylaniline (9.3 g, 14.2 mmol) obtained in the above-mentioned (1) was dissolved in n-heptane (64.9 g). A 4 mol/L hydrogen chloride/ethyl acetate solution (3.5 g, 15.8 mmol) was added dropwise thereto with stirring, and the mixture was stirred at room temperature for 2 hr. The resulting solid was collected by filtration, washed with n-heptane (12.8 g), and dried under reduced pressure to give the title compound (8.4 g, 12.2 mmol) .
   ¹H NMR(CDCl₃) δ: 13.71 (bs, 1H), 7.59 (d, J=8.6Hz, 2H), 7.29 (d, J=8.6hz, 2H), 3.32-3.54 (m, 2H), 3.02-3.22 (m, 2H), 2.65 (t, J=7.9Hz, 2H), 1.87-2.12 (m, 2H), 1.54-1.67 (m, 2H), 1.05-1.45 (m, 64H), 0.94 (t, J=7.3Hz, 3H), 0.87 (t, J=7.0Hz, 6H).

### [Production Example 3]

### Synthesis of N,N-dihexadecylaniline

To sodium hydride (content 66.4 wt%, 2.07 g, 55.5 mmol) was added tetrahydrofuran (10.72 g), and a solution of aniline (2.0 g, 21.9 mmol) in tetrahydrofuran (5.5 g) was added dropwise while heating with refluxing at 75°C in an oil bath. Then, to the reaction mixture was added dropwise a solution of 1-bromohexadecane (13.1 g, 42.9 mmol) in tetrahydrofuran (17.8 g), and the mixture was continuously heated under refluxed for 5 hr. The reaction mixture was cooled, and a mixed solution of water (0.4 g) and tetrahydrofuran (1.8 g) was added. After the hydrogen gas generation subsided, water (10.1 g) and tetrahydrofuran (6.3 g) were added thereto, and the mixture was separated. The organic layer was heated at 45°C, ethanol (111 g) was added, and the mixture was cooled to 20°C and stirred at 20°C to 25°C for 15 hr. The resulting solid was collected by filtration, washed with ethanol, and dried under reduced pressure to give the title compound (10.4 g).
¹H NMR(CDCl₃) δ: 7.12-7.23 (m, 3H), 6.55-6.69 (m, 2H), 3.24 (m, 4H), 1.58-1.49 (m, 4H), 1.30-1.11 (m, 52H), 0.88 (t, J=6.9 Hz, 6H) .

### [Production Example 4]

### Synthesis of N,N-dioctadecylaniline hydrochloride

To sodium hydride (content 66.4 wt%, 10.09 g, 270.7 mmol) was added tetrahydrofuran (53.6 g), and a solution of aniline (10.01 g, 107.5 mmol) in tetrahydrofuran (26.81 g) was added dropwise while heating with refluxing at 75°C in an oil bath. Then, to reaction mixture was added dropwise a solution of 1-bromooctadecane (78.9 g, 236.7 mmol) in tetrahydrofuran (89.1 g), and the mixture was continuously heated under refluxed for 5 hr. The reaction mixture was cooled, and a mixed solution of water (2.0 g) and tetrahydrofuran (8.9 g) was added. After the hydrogen gas generation subsided, water (50.0 g) was added thereto, and the mixture was separated. The organic layer was concentrated under reduced pressure, to the residue were added n-hexane (425 g) and water (50.7 g), and the mixture was stirred. To the organic layer were added n-hexane (43 g) and water (49.7 g), and the mixture was stirred again and separated to give a hexane solution (531.6 g) of N,N-dioctadecylaniline.

To the obtained hexane solution (528.1 g) of N,N-dioctadecylaniline was added n-hexane (170.7 g), 1 mol/L hydrogen chloride/diethyl ether solution (77.3 g, 118.7 mmol) was added dropwise with stirring, and the mixture was stirred at room temperature for 1.5 hr. The resulting solid was collected by filtration and washed with n-hexane (213 g). The solid was dispersed in n-hexane (637.3 g), stirred at 50°C for 2 hr, and cooled to room temperature. The solid was collected by filtration, washed with n-hexane (213 g), and dried under reduced pressure to give the title compound (65.9 g, 103.9 mmol).
¹H NMR(CDCl₃) δ: 7.19 (m, 2H), 6.64-6.59 (m, 3H), 3.40-3.05 (m, 4H), 1.57 (m, 4H), 1.02-1.46 (m, 60H), 0.88 (t, J=7.0Hz, 6H).

### [Production Example 5]

### Synthesis of N,N-dioctadecyl-4-methylaniline

To sodium hydride (content 66.4 wt%, 1.75 g, 47.0 mmol) was added tetrahydrofuran (10.7 g), and a solution of 4-methylaniline (2.0 g, 18.7 mmol) in tetrahydrofuran (5.4 g) was added dropwise while heating with refluxing at 75°C in an oil bath. Then, to the reaction mixture was added dropwise a solution of 1-bromooctadecane (12.4 g, 37.2 mmol) in tetrahydrofuran (17.85 g), and the mixture was continuously heated under refluxed for 5 hr. The reaction mixture was cooled, and a mixed solution of water (0.4 g) and tetrahydrofuran (1.9 g) was added. After the hydrogen gas generation subsided, water (10.0 g) was added thereto, and the mixture was separated. To the organic layer was added tetrahydrofuran (5.4 g), and ethanol (81 g) was added while stirring with heating at 45°C. The mixture was cooled to 25°C and continuously stirred for 20 hr. The resulting solid was collected by filtration, washed with ethanol, and dried under reduced pressure to give the title compound (10.2 g).
¹H NMR(CDCl₃) δ: 7.01 (d, J=8.2Hz, 2H), 6.56 (d, J=8.7Hz, 2H), 3.20 (t, J=7.8Hz, 4H), 2.23 (s, 3H), 1.47-1.65 (4H), 1.06-1.46 (m, 60H), 0.88 (t, J=6.9Hz, 6H).

### [Production Example 6]

### Synthesis of N,N-didodecyl-4-butylaniline hydrochloride

(1) To sodium hydride (content 66.4 wt%, 1.26 g, 33.8 mmol) was added tetrahydrofuran (10.8 g), and a solution of 4-butylaniline (2.0 g, 13.4 mmol) in tetrahydrofuran (5.5 g) was added dropwise while heating with refluxing at 75°C in an oil bath. Then, a solution of 1-bromododecane (6.7 g, 26.9 mmol) in tetrahydrofuran (17.9 g) was added dropwise to the reaction mixture, and the mixture was continuously heated under refluxed for 19 hr. The reaction mixture was cooled, and a mixed solution of water (0.4 g) and tetrahydrofuran (1.8 g) was added. After the hydrogen gas generation subsided, water (10.0 g) was added thereto, and the mixture was separated. The organic layer was concentrated under reduced pressure, the residue was dissolved in n-hexane (43.6 g), and the solution was washed with water, concentrated under reduced pressure, and dried to give N,N-didodecyl-4-butylaniline (6.7 g).
   ¹H NMR(CDCl₃) δ: 7.01 (d, J=8.7Hz, 2H), 6.57 (d, J=8.7Hz, 2H), 3.21 (t, J=7.8Hz, 4H), 2.49 (t, J=7.5Hz, 2H), 1.47-1.65 (m, 4H), 1.15-1.47 (m, 40H), 0.94-0.87 (m, 2H).
(2) N,N-Didodecyl-4-butylaniline (6.5 g, 13.4 mmol) obtained in the above-mentioned (1) was dissolved in n-hexane (21.6 g), and 4 mol/L hydrogen chloride/ethyl acetate solution (3.3 g, 15.0
mmol) was added dropwise with stirring, then n-hexane (6.4 g) was added, and the mixture was stirred at room temperature for 2 hr. The resulting solid was collected by filtration, washed with n-hexane (8.7 g), and dried under reduced pressure to give the title compound (5.8 g, 11.1 mmol).
¹H NMR(CDCl₃) δ: 13.70 (s, 1H), 7.59 (d, J=8.6Hz, 2H), 7.29 (d, J=8.6Hz, 2H), 3.31-3.58 (m, 2H), 2.87-3.28 (m, 2H), 2.65 (t, J=7.6Hz, 2H), 1.85-2.15 (m, 2H), 1.52-1.69 (m, 2H), 1.05-1.46 (m, 40H), 0.94 (t, J=7.3Hz, 3H), 0.87 (t, J=6.7Hz, 6H).

### [Production Example 7]

### Synthesis of N,N-ditetradecyl-4-butylaniline hydrochloride

(1) To sodium hydride (content 66.4 wt%, 1.26 g, 33.8 mmol) was added tetrahydrofuran (10.7 g, mmol), and a solution of 4-butylaniline (2.0 g, 13.4 mmol) in tetrahydrofuran (5.4 g) was added dropwise while heating with refluxing at 75°C in an oil bath. Then, a solution of 1-bromotetradecane (7.4 g, 26.7 mmol) in tetrahydrofuran (18.0 g) was added dropwise to the reaction mixture, and the mixture was continuously heated under refluxed for 12 hr. The reaction mixture was cooled, and a mixed solution of water (0.4 g) and tetrahydrofuran (1.9 g) was added. After the hydrogen gas generation subsided, water (10.0 g) was added thereto, and the mixture was separated. The organic layer was concentrated under reduced pressure, the residue was dissolved in n-hexane (53.6 g), and the solution was washed with water, concentrated under reduced pressure, and dried to give N,N-ditetradecyl-4-butylaniline (7.5 g).
   ¹H NMR(CDCl₃) δ: 7.01 (d, J=8.6Hz, 2H), 6.57 (d, J=9.2Hz, 2H), 3.20 (t, J=7.6Hz, 4H), 2.49 (t, J=7.9Hz, 2H), 1.46-1.65 (m, 4H), 1.00-1.46 (m, 48H), 0.78-0.98 (m, 9H).
(2) N,N-Ditetradecyl-4-butylaniline (7.0 g, 12.9 mmol) obtained in the above-mentioned (1) was dissolved in n-hexane (46 g), and 4 mol/L hydrogen chloride/ethyl acetate solution (3.1 g, 14.1 mmol) was added dropwise with stirring, and the mixture was stirred at room temperature for 1 hr. The resulting solid was collected by filtration, washed with n-hexane (23.3 g), and dried under reduced pressure to give the title compound (5.7 g, 9.9 mmol).

¹H NMR(CDCl₃) δ: 13.41-13.99 (bs, 1H), 7.59 (d, J=8.6Hz, 2H), 7.29 (d, J=7.9Hz, 2H), 3.29-3.55 (m, 2H), 3.01-3.28 (m, 2H), 2.65 (t, J=7.6Hz, 2H), 1.85-2.12 (m, 2H), 1.49-1.71 (m, 2H), 1.00-1.49 (m, 48H), 0.94 (t, J=7.3Hz, 3H), 0.87 (t, J=7.0Hz, 6H).

### [Example 1]

### N,N-Dihexadecyl-4-butylanilinium tetrakis(pentafluorophenyl)borate (compound (1-1))

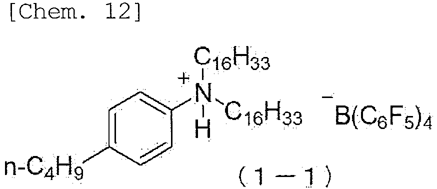

N,N-Dihexadecyl-4-butylaniline hydrochloride (5.0 g, 7.9 mmol) obtained in Production Example 1 was dissolved in methylcyclohexane (19.3 g), and water (10.0 g) and lithium tetrakis(pentafluorophenyl)borate tri-diethyl ether complex (8.2 g, 7.9 mmol) were added with stirring, and the mixture was stirred at room temperature for 2 hr. The reaction mixture was separated, the organic layer was washed with water, then methylcyclohexane (18 g) was evaporated under reduced pressure, methylcyclohexane (18 g) was added to the residue, and methylcyclohexane (16 g) was evaporated under reduced pressure. Methylcyclohexane (18 g) was again added to the residue, and methylcyclohexane (16 g) was evaporated under reduced pressure. Furthermore, methylcyclohexane (16 g) was again added to the residue, and methylcyclohexane (16 g) was evaporated under reduced pressure to give a methylcyclohexane solution of N,N-dihexadecyl-4-butylanilinium tetrakis(pentafluorophenyl)borate. As a result of quantitative analysis by NMR using an internal standard, the concentration of N,N-dihexadecyl-4-butylanilinium tetrakis(pentafluorophenyl)borate was 50.0 wt% (351 mM).

¹H NMR(CDCl₃) δ: 7.39 (d, J=8.2Hz, 2H), 7.02 (d, J=8.7Hz, 2H), 3.19-3.72 (m, 4H), 2.71 (t, J=7.8Hz, 2H), 1.55-1.74 (m, 2H), 1.00-1.55 (m, 58H), 0.96 (t, J=7.3Hz, 3H), 0.87 (t, J=6.9Hz, 6H);
¹⁹F NMR(CDCl₃) δ: -132.5 (d, J=10.1Hz, 8F), -161.8 (t, J=21.0Hz, 4F), -166.0 (t, J=18.1Hz, 8F).

### [Example 2]

### N,N-Dioctadecyl-4-butylanilinium tetrakis(pentafluorophenyl)borate (compound (1-2))

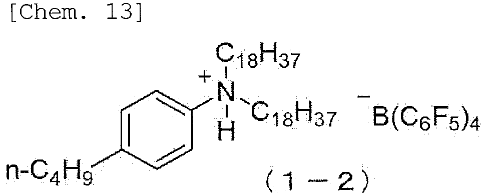

N,N-Dioctadecylaniline hydrochloride (5.0 g, 7.2 mmol) obtained in Production Example 2 was dissolved in methylcyclohexane (19.3 g), and water (10.0 g) and lithium tetrakis(pentafluorophenyl)borate tri-diethyl ether complex (8.2 g, 7.2 mmol) were added with stirring, and the mixture was stirred at room temperature for 2 hr. The reaction mixture was separated, the organic layer was washed with water, then methylcyclohexane (18 g) was evaporated under reduced pressure, methylcyclohexane (18 g) was added to the residue, and methylcyclohexane (16 g) was again evaporated under reduced pressure. Methylcyclohexane (18 g) was again added to the residue, and methylcyclohexane (16 g) was evaporated under reduced pressure. Furthermore, methylcyclohexane (16 g) was again added to the residue, and methylcyclohexane (16 g) was evaporated under reduced pressure to give a methylcyclohexane solution of the title compound. As a result of quantitative analysis by NMR using an internal standard, the concentration of N,N-dihexadecyl-4-butylanilinium tetrakis(pentafluorophenyl)borate was 50.9 wt% (333 mM).
¹H NMR(CDCl₃) δ: 0.87 (d, J=6.9Hz, 6H), 0.96 (t, J=7.3Hz, 3H), 1.01-1.53 (m, 66H), 1.57-1.73 (m, 2H), 2.71 (t, J=7.8Hz, 2H), 3.22-3.73 (m, 4H), 7.02 (d, J=8.7Hz, 2H), 7.39 (d, J=8.7Hz, 2H); ¹⁹F NMR(CDCl₃) δ: -132.5 (d, J=10.1Hz, 8F), -161.8 (t, J=21.0Hz, 4F), -166.0 (t, J= 18.1Hz, 8F).

### [Comparative Example 1]

### N,N-Dihexadecylanilinium tetrakis(pentafluorophenyl)borate (compound of Comparative Example 1)

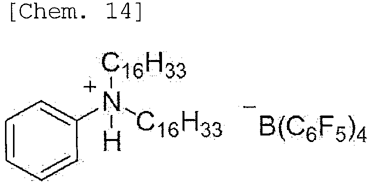

N,N-Dihexadecylaniline (6.0 g, 11.1 mmol) obtained in Production Example 3 was dissolved in methylcyclohexane (24.6 g), and water (7.2 g) and 2 mol/L hydrochloric acid (5.8 g, 11.3 mmol) were added with stirring. To the reaction mixture were added lithium tetrakis(pentafluorophenyl)borate tri-diethyl ether complex (11.5 g, 11.1 mmol), and the mixture was stirred at room temperature for 2 hr. The obtained reaction mixture was separated, the organic layer was washed with water, then methylcyclohexane (23 g) was evaporated under reduced pressure, methylcyclohexane (24 g) was added to the residue, and methylcyclohexane (24 g) was again evaporated under reduced pressure. The same addition and evaporation of methylcyclohexane was repeated two more times to give a methylcyclohexane solution of the title compound (52.7 wt% (401 mM)).
¹H NMR(CDCl₃) δ: 7.54-7.71 (m, 3H), 7.07-7.21 (m, 2H), 3.51 (t, J=8.0Hz, 4H), 0.99-1.52 (56H), 0.87 (t, J=6.9hz, 6H);
¹⁹F NMR(CDCl₃) δ: -132.5 (d, J=10.1Hz, 8F), -161.7 (t, J=21.0Hz, 4F), -166.0 (t, J=18.1Hz, 8F).

### [Comparative Example 2]

### N,N-Dioctadecylanilinium tetrakis(pentafluorophenyl)borate (compound of Comparative Example 2)

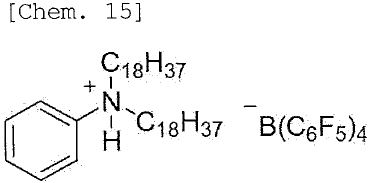

N,N-Dioctadecylaniline hydrochloride (7.02 g, 11.1 mmol) obtained in Production Example 4 and methylcyclohexane (27.1 g) were mixed, lithium tetrakis(pentafluorophenyl)borate tri-diethyl ether complex (11.4 g, 11.0 mmol) and water (14.0 g) were added with stirring, and the mixture was stirred at room temperature for 2 hr. The obtained reaction mixture was separated, and the organic layer was washed with water, then methylcyclohexane (26 g) was evaporated under reduced pressure, methylcyclohexane (27 g) was added to the residue, and methylcyclohexane (22 g) was again evaporated under reduced pressure. The same addition and evaporation of methylcyclohexane was repeated two more times to give a methylcyclohexane solution of the title compound. As a result of quantitative analysis by NMR using an internal standard, the concentration of N,N-dioctadecylanilinium tetrakis(pentafluorophenyl)borate in the solution was 49.0 wt% (338 mM).
¹H NMR(CDCl₃) δ: 7.54-7.70 (m, 3H), 7.15 (d, J=6.7Hz, 2H), 3.51 (t, J=7.9Hz, 4H), 1.35-1.59 (m, 4H), 0.94-1.35 (60H), 0.87 (t, J=6.7Hz, 6H);
¹⁹F NMR(CDCl₃) δ: -132.6 (d, J=10.1Hz, 8F), -161.8 (t, J=21.0Hz, 4F), -166.0 (t, J=18.1Hz, 8F).

### [Comparative Example 3]

### N,N-Dioctadecyl-4-methylanilinium tetrakis(pentafluorophenyl)borate (compound of Comparative Example 3)

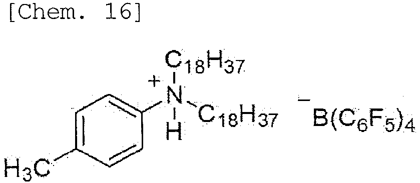

N,N-Dioctadecyl-4-methylaniline (5.0 g, 8.2 mmol) obtained in Production Example 5 was dissolved in methylcyclohexane (20.5 g), and water (6.5 g) and 2 mol/L hydrochloric acid (4.3 g, 8.3 mmol) were added with stirring. To the reaction mixture was added lithium tetrakis(pentafluorophenyl)borate tri-diethyl ether complex (8.5 g, 8.2 mmol), and the mixture was stirred at room temperature for 2 hr. The obtained reaction mixture was separated, and the organic layer was washed with water, then methylcyclohexane (17 g) was evaporated under reduced pressure, methylcyclohexane (17 g) was added to the residue, and methylcyclohexane (14 g) was again evaporated under reduced pressure. Methylcyclohexane (15 g) was again added to the residue, and methylcyclohexane (16 g) was evaporated under reduced pressure to give a methylcyclohexane solution of the title compound. As a result of quantitative analysis by NMR using an internal standard, the concentration of N,N-dihexadecyl-4-methylanilinium tetrakis(pentafluorophenyl)borate in the solution was 51.3 wt% (366 mM).
¹H NMR(CDCl₃) δ: 7.38 (d, J=8.2Hz, 2H), 7.00 (d, J=8.7Hz, 2H), 3.47 (t, J=8.0Hz, 4H), 2.46 (s, 3H), 0.95-1.54 (64H), 0.87 (t, J=6.9Hz, 6H);
¹⁹F NMR(CDCl₃) δ: -132.5 (d, J=10.1Hz, 8F), -161.8 (t, J=20.2Hz, 4F), -166.0 (t, J=18.1Hz, 8F).

### [Comparative Example 4]

### N,N-Didodecyl-4-butylanilinium tetrakis(pentafluorophenyl)borate (compound of Comparative Example 4)

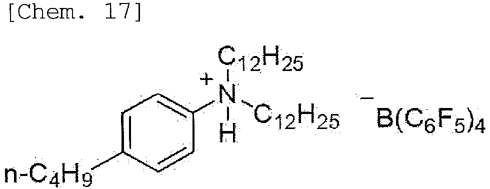

N,N-Didodecyl-4-butylaniline hydrochloride (5.0 g, 9.6 mmol) obtained in Production Example 6 was dissolved in methylcyclohexane (19.3 g), water (10.0 g) and lithium tetrakis(pentafluorophenyl)borate tri-diethyl ether complex (9.9 g, 9.6 mmol) were added with stirring, and the mixture was stirred at room temperature for 2 hr. The obtained reaction mixture was separated, and the organic layer was washed with water, then methylcyclohexane (12 g) was evaporated under reduced pressure, methylcyclohexane (12 g) was added to the residue, and methylcyclohexane (10 g) was again evaporated under reduced pressure. Methylcyclohexane (21 g) was again added to the residue, and methylcyclohexane (9 g) was evaporated under reduced pressure to give a methylcyclohexane solution of the title compound. As a result of quantitative analysis by NMR using an internal standard, the concentration of N,N-didodecyl-4-butylanilinium tetrakis(pentafluorophenyl)borate in the solution was 58.6 wt% (472 mM).
¹H NMR(CDCl₃) δ: 7.39 (d, J=8.7Hz, 2H), 7.02 (d, J=8.7Hz, 2H), 3.48 (bs, 4H), 2.71 (t, J=8.0Hz, 2H), 1.54-1.75 (m, 2H), 0.99-1.54 (m, 42H), 0.96 (t, J=7.3Hz, 3H), 0.88-0.84 (t, J=6.9Hz, 6H);
¹⁹F NMR(CDCl₃) δ: -132.5 (d, J=10.1Hz, 8F), -161.8 (t, J=20.2Hz, 4F), -166.0 (t, J=18.8Hz, 8F).

### [Comparative Example 5]

### N,N-Ditetradecyl-4-butylanilinium tetrakis(pentafluorophenyl)borate (compound of Comparative Example 5)

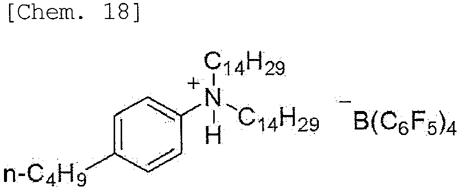

N,N-Ditetradecyl-4-butylaniline hydrochloride (5.0 g, 8.6 mmol) obtained in Production Example 7 was dissolved in methylcyclohexane (19.3 g), water (10.0 g) and lithium tetrakis(pentafluorophenyl)borate tri-diethyl ether complex (9.0 g, 8.7 mmol) were added with stirring, and the mixture was stirred at room temperature for 2 hr. The obtained reaction mixture was separated, and the organic layer was washed with water, then methylcyclohexane (17 g) was evaporated under reduced pressure, methylcyclohexane (17 g) was added to the residue, and methylcyclohexane (16 g) was again evaporated under reduced pressure. Methylcyclohexane (18 g) was again added to the residue, and methylcyclohexane (16 g) was evaporated under reduced pressure. Furthermore, methylcyclohexane (17 g) was again added to the residue, and methylcyclohexane (17 g) was evaporated under reduced pressure to give a methylcyclohexane solution of the title compound. As a result of quantitative analysis by NMR using an internal standard, the concentration of N,N-ditetradecyl-4-butylanilinium tetrakis(pentafluorophenyl)borate in the solution was 55.5 wt% (423 mM).
¹H NMR(CDCl₃) δ: 7.38 (d, J=8.7Hz, 2H), 7.02 (d, J=8.7Hz, 2H), 3.48 (bs, 4H), 2.71 (t, J=7.8Hz, 2H), 1.54-1.74 (m, 2H), 1.01-1.54 (m, 50H), 0.95 (t, J=7.3Hz, 3H), 0.86 (t, J=6.9Hz, 6H);
¹⁹F NMR(CDCl₃) δ: -132.5 (d, J=10.1Hz, 8F), -161.8 (t, J=20.2Hz, 4F), -166.0 (t, J=18.1Hz, 8F).

### [Experimental Example 1] (Evaluation of solubility)

Methylcyclohexane solutions containing 50% by weight of each of the compounds obtained in Examples 1 and 2 and Comparative Examples 1 to 5, and n-hexane solutions containing 0.6% by weight (≈ 3 mM) of each of the aforementioned compounds were prepared and stored at room temperature or -15°C for 3 days, and the appearance of each solution was confirmed by visual observation. The results are shown in Table 1 and Table 2 below. The symbols used to evaluate solubility in the Tables mean the following:
O: homogeneous transparent solution state
Δ: cloudy state
×: two-phase separation or crystallization

**[Table 1]**

| Solubility evaluation results of 50 wt% borate compound/methylcyclohexane solution | | | |
|---|---|---|---|
| borate compound | concentration [mM] (50 wt%) | solubility room temperature, 3 days | solubility -15°C, 3 days |
| Example 1 (compound (1-1)) | 356 | ○ | ○* |
| Example 2 (compound (1-2)) | 342 | ○ | ○ |
| Comparative Example 1 | 404 | ○ | ○ |
| Comparative Example 2 | 351 | ○ | ○ |
| Comparative Example 3 | 348 | ○ | ×** |
| Comparative Example 4 | 394 | ○ | ○ |
| Comparative Example 5 | 368 | ○ | ○ |

| | | | |
|---|---|---|---|
| * homogeneous transparent solution even at -20°C (○) ** two-phase separation or crystallization even at -20°C (×) | | | |

**[Table 2]**

| Solubility evaluation results of 0.6 wt% (≒ 3 mM) borate compound/n-hexane solution | | |
|---|---|---|
| borate compound | solubility room temperature, 3 days | solubility -15°C, 3 days |
| Example 1 (compound (1-1)) | ○ | ○ |
| Example 2 (compound (1-2)) | ○ | ○ |
| Comparative Example 1 | ○ | × |
| Comparative Example 2 | ○ | × |
| Comparative Example 3 | ○ | × |
| Comparative Example 4 | ○ | × |
| Comparative Example 5 | ○ | × |

According to Tables 1 and 2, it was confirmed that Examples 1 and 2 can maintain remarkably high solubility in aliphatic hydrocarbon solvents even at low temperatures compared to Comparative Examples 1 to 5.

### [Experimental Example 2] (Evaluation of polymerization performance)

A general polymerization method using the compound of the present invention (compound (1-2)) as a cocatalyst is shown below.

Into 100 mL autoclave in a glove box were added 1-octene, triisobutylaluminum (TIBA, 1.00 M hexane solution) and a solvent (methylcyclohexane (MCH)) to prepare a comonomer solution. A polymerization catalyst (dimethylsilylene(tert-butylamide)-(tetramethylcyclopentadienyl)-titanium (IV)-dichloride (CGC)), triisobutylaluminum (1.00 M hexane solution), and a solvent were added to prepare a catalyst solution at a predetermined concentration, and the solution was transferred to a Schlenk tube. Compound (1-2) (cocatalyst) was dissolved in a solvent, and a cocatalyst solution at a predetermined concentration was prepared and transferred to the Schlenk tube. The comonomer solution, the catalyst solution, and the cocatalyst solution were mixed, and prepared such that the total amount of the solvent and the total amount of triisobutylaluminum would be constant. The inside of the autoclave was purged with ethylene gas, the catalyst solution and the cocatalyst solution were successively added to the autoclave, and the ethylene gas pressure was immediately adjusted to a predetermined pressure, and the mixture was stirred at 50°C for 6 min. The reaction mixture was ice-cooled, the ethylene gas was removed, the mixture was poured into methanol (100 mL) containing hydrochloric acid (3 mL), and the mixture was stirred at room temperature for 30 min. The precipitate was collected by filtration and dried under reduced pressure at 60°C to give an ethylene-octene copolymer (47.3 mg).

### (Reaction conditions)

Catalyst: CGC (3 µmol);
catalyst: cocatalyst=1:1;
TIBA (3000 µmol);
Total amount of solvent (40 ml);
1-octene (1 mL);
Ethylene pressure (8 atm);
Reaction temperature 50°C;
Reaction time 6 min

### (Melting point measurement)

The melting point of the sample was measured by differential scanning calorimetry (DSC). It was performed using DSC7000X (manufactured by Hitachi High-Tech Science Corporation). To remove thermal history, the sample was heated from room temperature to 250°C (heating rate: 20°C/min) and then maintained at 250°C for 5 min. Thereafter, the temperature was lowered to -100°C at 10°C/min, maintained for 10 min, and then heated from -100°C to 250°C at a rate of 10°C/min. The melting point during the second heating was recorded. The melting point of the polymer obtained under the aforementioned conditions was 127.5°C.

As mentioned above, it was clarified that the compound of the present invention functions as a cocatalyst for metallocene catalyzed polymerization.

### [Industrial Applicability]

According to the present invention, a novel borate compound capable of maintaining a homogeneous solution state in an aliphatic hydrocarbon solvent, particularly n-hexane, methylcyclohexane, or a mixed solvent thereof, even at low temperatures, can be provided without using any aromatic hydrocarbon solvent. The compound of the present invention can maintain a homogeneous solution state in aliphatic hydrocarbon solvents even at low temperatures, and thus affords the advantage that the compound of the present invention can be stably stored in a diluted solution state that facilitates handling and measurement, and can be used as it is when in use as a cocatalyst for solution polymerization reaction of olefin and diene. According to the present invention, moreover, a convenient production method of the compound of the present invention can also be provided.

This application is based on a patent application No. 2023-156460 filed in Japan (filing date: September 21, 2023), the contents of which are incorporated in full herein.

## Claims

1. A compound represented by the formula (1): 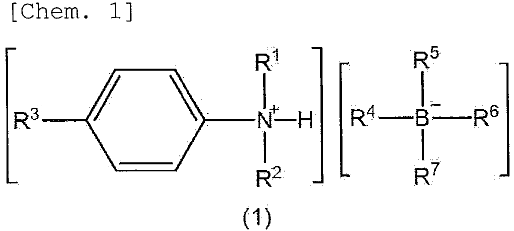
wherein R¹ and R² are each independently a linear C₁₆₋₂₀ alkyl group; R³ is a linear C₃₋₈ alkyl group; and
R⁴, R⁵, R⁶ and R⁷ are each independently a C₆₋₁₄ aryl group substituted by one or more fluorine atoms or fluoro C₁₋₄ alkyl groups.

2. The compound according to claim 1, wherein R¹ and R² are each independently a linear C₁₆₋₁₈ alkyl group, and R³ is a linear C₄₋₆ alkyl group.

3. The compound according to claim 1, wherein R⁴, R⁵, R⁶ and R⁷ are each independently a phenyl group, a 1-naphthyl group, or a 2-naphthyl group, each of which is substituted by one or more fluorine atoms or trifluoromethyl groups.

4. The compound according to claim 1, wherein all of R⁴, R⁵, R⁶ and R⁷ are pentafluorophenyl groups, 2,3,4,5,6,7,8-heptafluoro-1-naphthyl groups, or 1,3,4,5,6,7,8-heptafluoro-2-naphthyl groups.

5. The compound according to any one of claims 1 to 4, wherein the compound exhibits a solubility of 3 mM or more in n-hexane within the range of -15°C to 25°C and a solubility of 300 mM or more in methylcyclohexane within the range of -15°C to 25°C.

6. A n-hexane solution of the compound according to any one of claims 1 to 4, wherein 3 mM or more of the compound is contained within the range of -15°C to 25°C.

7. A methylcyclohexane solution of the compound according to any one of claims 1 to 4, wherein 300 mM or more of the compound is contained within the range of -15°C to 25°C.

8. A cocatalyst for solution polymerization of at least one kind of monomer selected from the group consisting of an olefin and a diene, comprising the compound according to any one of claims 1 to 4.

9. A method for storing the compound according to any one of claims 1 to 4, comprising dissolving the compound in n-hexane or methylcyclohexane, and storing same at -15°C to 0°C while maintaining a homogeneous solution state.

10. The method for producing the compound according to claim 1, comprising a step of reacting a compound represented by the formula (2): 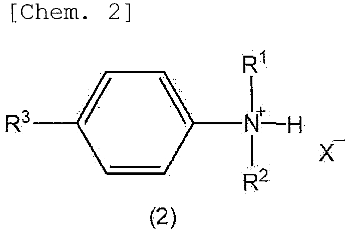
wherein R¹ and R² are each independently a linear C₁₆₋₂₀ alkyl group; R³ is a linear C₃₋₈ alkyl group; and
X is a halogen atom, and a compound represented by the formula (3): 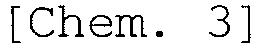 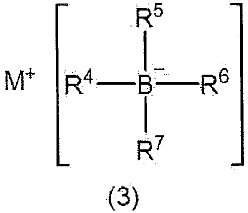
wherein R⁴, R⁵, R⁶, and R⁷ are each independently a C₆₋₁₄ aryl group substituted by one or more fluorine atoms or fluoro C₁₋₄ alkyl groups; and
M is an alkali metal, in a mixed solvent of methylcyclohexane and water.
